# EUROPEAN PATENT SPECIFICATION

(11) **EP 3 095 438 B1**
(45) Date of publication and mention of the grant of the patent: **30.08.2017**
(21) Application number: 16178588.6
(22) Date of filing: 20.11.2014
(51) Int. Cl.: A61K 8/64, A61Q 19/02, A61K 8/11, A61K 8/81, A61K 8/85, C07K 14/415

(54) **NEW PEPTIDES FOR THE PREVENTION AND/OR TREATMENT OF SKIN DARK SPOTS**
NEUE PEPTIDE ZUR PRÄVENTION UND/ODER BEHANDLUNG VON DUNKLEN FLECKEN AUF DER HAUT
NOUVEAUX PEPTIDES POUR LA PRÉVENTION ET/OU LE TRAITEMENT DE TACHES SOMBRES SUR LA PEAU

(30) Priority: 20.11.2013 EP 13382467
(43) Date of publication of application: 23.11.2016
(62) Divisional of application: 14800064.9
(73) Proprietor: Infinitec Activos S.L., 08170 Montornés del Vallès, Barcelona (ES)
(72) Inventor: MOURELLE MANCINI, Marisabel, E-08028 Barcelona (ES); CARCELLER MARGELI, Magdalena, E-08907 Hospitalet de Llobregat, Barcelona (ES)
(74) Representative: ABG Patentes, S.L.

(56) References cited:
- WO-A1-2012/013136
- WO-A1-2013/081147
- CN-B- 101 865 925
- JP-A- 2006 062 991
- KR-A- 20100 097 965

### SEQUENCE LISTING

<110> INFINITEC ACTIVOS S.L.
<120> NEW PEPTIDES FOR THE PREVENTION AND/OR TREATMENT OF SKIN DARK SPOTS
<130> P10071EPPC01
<150> EP14800064.9
   <151> 2014-11-20
<160> 4
<170> PatentIn version 3.5
<210> 1
   <211> 13
   <212> PRT
   <213> Homo sapiens
<220>
   <221> MOD_RES
   <222> (1)..(1)
   <223> ACETYLATION
<220>
   <221> MOD_RES
   <222> (13)..(13)
   <223> AMIDATION
<400> 1
   Ser Tyr Ser Met Glu His Phe Arg Trp Gly Lys Pro Val
   1               5                   10
<210> 2
   <211> 8
   <212> PRT
   <213> Artificial Sequence
<220>
   <223> Tyrosinase inhibitor peptide
<400> 2
   Ile Ser Leu Leu Asp Ala Gln Ser
   1               5
<210> 3
   <211> 8
   <212> PRT
   <213> Artificial Sequence
<220>
   <223> Tyrosinase inhibitor peptide
<220>
   <221> MOD_RES
   <222> (1)..(1)
   <223> ACETYLATION
<220>
   <221> MOD_RES
   <222> (8)..(8)
   <223> AMIDATION
<400> 3
   Ile Ser Leu Leu Asp Ala Gln Ser
   1               5
<210> 4
   <211> 8
   <212> PRT
   <213> Artificial Sequence
<220>
   <223> Tyrosinase inhibitor peptide
<220>
   <221> LIMPID
   <222> (1)..(1)
   <223> PALMITATE
<220>
   <221> MOD_RES
   <222> (8)..(8)
   <223> AMIDATION
<400> 4
   Ile Ser Leu Leu Asp Ala Gln Ser
   1               5

## Description

### FIELD OF THE INVENTION

The present invention relates to the field of pharmacy and cosmetics, in particular, it relates to new peptides for the cosmetic use for the prevention and/or treatment of skin dark spots.

### BACKGROUND

In dermatology, hyperpigmentation is the darkening of an area of skin or nails caused by an increased or excess production of melanin. Melanin is produced by melanocytes at the lower layer of the epidermis. The production of melanin is called melanogenesis. There are three basic types of melanin: eumelanin, pheomelanin, and neuromelanin.

Melanocortin receptor 1 (MC1 R) is known as a peripheral receptor that is mainly found in different skin cells (melanocytes, keratinocytes, sebocytes, and others). MC1R is involved in pigmentation and inflammation responses, and on various melanoma cells, and found to control the relative amounts of eumelanin and pheomelanin in mammals. (Bednarek, M. et al., Biopolymers, 2008, 89(5), 401-408).

α-Melanocyte Stimulating Hormone (α-MSH): Ac-Ser¹-Tyr¹-Ser³-Met⁴-Glu⁵-His⁶-Phe⁷-Ar⁸-Tr⁹-Gly¹⁰-Lys¹¹-Pro¹²-Val¹³-NH₂ (SEQ ID NO: 1) is an endogenous agonist for the MC1 R.

From previous studies with α-MSH and its synthetic analogue (NDP-α-MSH): Ac-Ser¹-Tyr²-Ser³-Nle⁴-Glu⁵-His⁶-D-Phe⁷-Arg⁸-Trp⁹-Gly¹⁰-Lys¹¹-Pro¹²-Val¹³-NH₂ it has been considered that segment 6-9 (His⁶-D-Phe⁷-Arg⁸-Trp⁹) is essential for the ligand-receptor interaction.

Analogues of the previous mentioned peptides were synthesized with the following various amino acids in place of Phe⁷ and Trp⁹ in NDP-α-MSH: Ala, Val, Cha, Lys, Glu, Phe, Pip, Pro, Oic, Tic, Sar, Gly. In view of the results Bednarek et al conclude that Trp⁹ residue of NDP-αMSH is not essential for molecular recognition at human melanocortin receptor 1 (hMC1bR).

WO 2008/107533 discloses a combination of at least one MC1 R receptor inhibitor, a tyrosinase inhibitor derived from vitamin C and an inhibitor of the transfer of melanosomes to the keratinocytes, to improve the activity of the tyrosinase inhibitor in the treatment of dark spots on the skin. It also describes the use of the combination in cosmetics and dermatology for preparing whitening and/or bleaching depigmentation compositions.

Among the MC1-R receptor inhibitors WO 2008/107533 lists the Agouti protein, Melanostatine ®5 (INCI name: nonapeptide-1) and the lipo amino acid undecylenoyl phenylalanine commercialized under the name Sepiwhite MSH ®.

Melanostatine ®5 consist in a synthetic nonapeptide of the following amino acids: Arg-Lys-Met-D-Phe-Phe-Pro-Pro-Trp-Val.

Sepiwhite is the commercial name of N-undecyl-10-enoyl-L-phenylalanine. Sepiwhite is a reported alpha-melanocyte-stimulating hormone (α-MSH) receptor antagonist which reduces melanin production in cultured melanocytes. Other peptides with whitening effects are disclosed in WO2012/013136, WO2013/081147, KR2010/0097965, JP2006/062991 and CN101865925. Among the tyrosinase inhibitors derived from vitamin C WO 2008/107533 cites the esthers of ascorbic acid, for example, the esther of 2-glucoside ascorbic acid (INCI name: Ascorbyl Glucoside, AA-2G), 2-O-α-D-glucopyranosyl-6-O-hexadecanoyl-L-ascorbic acid, ascorbyl 6-palmitate, sodium or magnesium 2-phosphate ascorbic acid salt.

As inhibitors of the transfer of melanosomes, WO 2008/107533 describes the Nicotinamide (INCI name: niacinamide) and PP vitamin. It particularly describes a composition comprising niacianimide (0.01%-5%), ascorbyl glucoside (0.01%-5%) and melanostatine ® (0.1-40 ppm).

It has been reported that the combination of N-undecyl-10-enoyl-L-phenylalanine (Sepiwhite) and niacinamide, is significantly more effective than the niacinamide alone in reducing facial hyperpigmentation. Niacinamide has been found to inhibit melanosome transfer in cultured cells and to reduce the appearance of hyperpigmented spots in clinical studies. (Bissett, D.L.,J. Cosmet Dermatol, 2009; 8(4) 260-6).

As shown in the art discussed above there is a continuous interest in finding new ingredients which are useful in the prevention and/or treatment of skin dark spots.

### BRIEF DESCRIPTION OF THE INVENTION

The inventors have surprisingly found a new peptide of formula (II):

R₅-Ile-Ser-Leu-Leu-Asp-Ala-Gln-Ser-R₆ [R₅-(SEQ ID NO: 2)-R₆] (II)

wherein
R₅ is selected from the group consisting of H-, R₇-C(O)- and R₇-OC(O)-,
R₆ is selected from the group consisting of -OH, -OR₇, -SR₇, and -NR₇R₈,
R₇ and R₈ are independently selected from H-, C₁-C₂₄ alkyl, C₂-C₂₄ alkenyl and C₆-C₁₀ aryl,
and cosmetically acceptable salts and solvates thereof.

Said peptide is useful in the cosmetic prevention and/or cosmetic treatment of cutaneous dark spots. Thus, another aspect refers to the use of the peptide of formula (II) as defined above for the cosmetic prevention and/or cosmetic treatment of cutaneous dark spots.

Another aspect of this invention refers to compositions comprising a peptide of formula (II) and one or more whitening agent such as arbutine, vitamin C, ascorbyl phosphate, niacinamide, dynachondrine, a tyrosinase inhibitor peptide, tretinoin, hydroquinone, kojic acid and esters thereof with fatty acids, azelaic acid, glutathione, cinnamomum subavenium extracts, alpha-hydroxy acids such as lactic acid or glycolic acid, niacinamide, ferulic acid, vitamin E, ellagic acid, pomegrate extracts and mixtures thereof.

These aspects and preferred embodiments thereof are described below. The invention is defined by the appended claims.

### DETAILED DESCRIPTION OF THE INVENTION

In the context of the present invention, the following terms have the meaning detailed below.

In the context of the present invention whenever an aminoacid is mentioned without specifying its stereochemistry it is to be understood that the mention designates both the D and the L stereosiomers. For example, any mention to Leucine (or Leu) designates both D-Leucine and L-Leucine. When a specific stereoisomery is intented, this is indicated, for example D-Ala is used to designated the D stereoisomer of Alanine.

"Alkyl" groups may be branched or unbranched, and preferably have from 1 to about 24 carbon atoms. One more preferred class of alkyl groups has from 1 to about 16 carbon atoms and a more preferred class of alkyl groups has from 1 to 6 and 14 to 18. Even more preferred are alkyl groups having 1, 2, 3, 4, 15, 16 or 17 carbon atoms. Methyl, ethyl, n-propyl, isopropyl and butyl, including n-butyl, tert-butyl, sec-butyl, isobutyl, pentadecyl, hexadecyl, heptadecyl are particularly preferred alkyl groups in the compounds of the present invention. Another preferred class of alkyl groups has from 6 to about 10 carbon atoms; and even more preferably 7, 8 or 9 carbon atoms. Heptyl, octyl and nonyl are the most preferred alkyl groups of this class.

The term "C₂-C₁₂ alkenyl" means a linear or branched hydrocarbon chain radical having one or more carbon-carbon double bonds therein and having from two to twelve carbon atoms, and which is attached to the rest of the molecule by a single bond. The double bond of an alkenyl group can be unconjugated or conjugated to another unsaturated group. Suitable alkenyl groups include, but are not limited to alkenyl groups such as vinyl, allyl, butenyl (e.g. 1-butenyl, 2-butenyl, 3-butenyl), pentenyl (e.g. 1-pentenyl, 2-pentenyl, 3-pentenyl, 4-pentenyl,), hexenyl (e.g. 1-hexenyl, 2-hexenyl, 3-hexenyl, 4-hexenyl, 5-hexenyl,), butadienyl, pentadienyl (e.g. 1,3-pentadienyl, 2,4-pentadienyl), hexadienyl (e.g. 1,3-hexadienyl, 1,4-hexadienyl, 1,5-hexadienyl, 2,4-hexadienyl, 2,5-hexadienyl), 2-ethylhexenyl (e.g. 2-ethylhex-1-enyl, 2-ethylhex-2-enyl, 2-ethylhex-3-enyl, 2-ethylhex-4-enyl, 2-ethylhex-5-enyl,), 2-propyl-2-butenyl, 4,6-dimethyl-oct-6-enyl. An alkenyl group can be unsubstituted or substituted with one or two suitable substituents.

"Aryl" herein refers to single and double ring radicals from 6 to about 10 carbon ring atoms, such as phenyl, naphthyl or indenyl. The aryl radical may be optionally substituted by one or more substituents such as hydroxy, mercapto, halo, alkyl, phenyl, alkoxy, haloalkyl, nitro, cyano, dialkylamino, aminoalkyl, acyl, alkoxycarbonyl, etc.

The cosmetically acceptable salts of the peptides provided by this invention are also within the scope of the present invention. The term "cosmetically acceptable salts" means a salt generally admitted for its use in animals and more particularly in human beings, and includes the salts used to form base addition salts, either inorganic base addition salts, such as for example and in a non-limiting sense, lithium, sodium, potassium, calcium, magnesium or aluminium among others, or organic base addition salts, such as for example and in a non-limiting sense ethylamine, diethylamine, ethylenediamine, ethanolamine, diethanolamine, arginine, lysine, histidine or piperazine among others, or acid addition salts, either organic acid addition salts, such as for example and in a non-limiting sense acetate, citrate, lactate, malonate, maleate, tartrate, fumarate, benzoate, aspartate, glutamate, succinate, oleate, trifluoroacetate, oxalate, pamoate or gluconate among others, or inorganic acid addition salts, such as for example and in a non-limiting sense chloride, sulfate, borate or carbonate among others. The nature of the salt is not critical, provided that it is cosmetically acceptable. The cosmetically acceptable salts of the peptide derivatives of the invention can be obtained by conventional methods well known in the state of the art [Berge S.M., Bighley L.D. and Monkhouse D.C. (1977) "Pharmaceutical Salts" J. Pharm. Sci. 66:1-19].

The term "skin whitening compound" or "skin whitening agent" refers to a compound able to light and/or delete dark spots on the skin. The present invention refers as whitening compounds to compounds that inhibit the activity or expression of tirosinase, i.e. in an embodiment of the present invention a "skin whitening compound" is a "tirosinase inhibitor". In one embodiment the skin whitening compound is selected from the group consisting of arbutin, vitamin C, sodium ascorbyl phosphate, niacinamide, preferably niacinamide PC, a tyrosinase inhibitor peptide and mixtures thereof. In a preferred embodiment the skin whitening compound is selected from the group consisting of arbutin, sodium ascorbyl phosphate, vitamin C, niacinamide PC, a tyrosinase inhibitor peptide, tretinoin, hydroquinone, kojic acid and esters thereof with fatty acids, azelaic acid, glutathione, cinnamomum subavenium extracts, alpha-hydroxy acids such as lactic acid or glycolic acid, niacinamide, ferulic acid, vitamin E, ellagic acid, pomegrate extracts and mixtures thereof.

In a particular embodiment the mixture of skin whitening compounds is selected from mixture of two skin whitening compounds, more preferably the mixture of skin whitening compounds is selected from a mixture of arbutin and sodium ascorbyl phosphate and a mixture of niacinamide and sodium ascorbyl phosphate.

In one aspect the present invention relates to a tyrosinase inhibitor peptide of formula (II). The peptide of formula (II) acts intracellularly blocking or inhibiting the activity of the tirosinase

Preferably the tyrosinase inhibitor peptide is a peptide of formula (II):

R₅-Ile-Ser-Leu-Leu-Asp-Ala-Gln-Ser-R₆ R₅-[(SEQ ID NO: 2)-R₆] (II)

wherein
R₅ is selected from the group consisting of H-, R₇-C(O)- and R₇-OC(O)-,
R₆ is selected from the group consisting of -OH, -OR₇, -SR₇, and -NR₇R₈,
R₇ and R₈ are independently selected from H-, C₁-C₂₄ alkyl, C₂-C₂₄ alkenyl and C₆-C₁₀ aryl,
and cosmetically acceptable salts and solvates thereof.

In one embodiment the tyrosinase inhibitor peptide is a peptide of formula:

R₅-Ile-Ser-D-Leu-Leu-Asp-D-Ala-Gln-Ser-R₆

wherein
R₅ is selected from the group consisting of H-, R₇-C(O)- and R₇-OC(O)-,
R₆ is selected from the group consisting of -OH, -OR₇, -SR₇, and -NR₇R₈,
R₇ and R₈ are independently selected from H-, C₁-C₂₄ alkyl, C₂-C₂₄ alkenyl and C₆-C₁₀ aryl,
and cosmetically acceptable salts and solvates thereof

In one embodiment R₇ and R₈ are H.

In one embodiment R₇ is C₁-C₂₄ alkanoyl, prefereably a C₁-C₂₄ alkanoyl selected from the group consisting of acetyl, propanoyl, butanoyl, pentanoyl, hexanoyl, heptanoyl, octanoyl, nonanoyl, decanoyl, undecanoyl, dodecanoyl, tridecanoyl, tetradecanoyl, pentadecanoyl, hexadecanoyl, heptadecanoyl, octadecanoyl, nonadecanoyl and icosanoyl. In a preferred embodiment the alkanoyl selected from the group consisting of acetyl, propanoyl, pentadecanoyl, hexadecanoyl and heptadecanoyl. In one embodiment R₅ is acetyl (Ac). In another embodiment R₅ is hexadecanoyl (or commonly named palmitoyl (Palm)).

In one embodiment, when R₆ is -NR₇R₈, R₇ and R₈ are H.

In one embodiment the peptide of formula (II) is selected from the group:
Ac-Ile-Ser-DLeu-Leu-Asp-DAla-Gln-Ser-NR₇R₈
Ac-Ile-Ser-Leu-Leu-Asp-DAla-Gln-Ser-NR₇R₈
Ac-Ile-Ser-Leu-Leu-Asp-Ala-Gln-Ser-NR₇R₈ [(SEQ ID NO: 3)-NR₇R₈]
Palm-Ile-Ser-Leu-Leu-Asp-DAla-Gln-Ser-NR₇R₈
Palm-Ile-Ser-Leu-Leu-Asp-Ala-Gln-Ser-NR₇R₈[(SEQ ID NO: 4)-NR₇R₈]
Palm-Ile-Ser-DLeu-Leu-Asp-DAla-Gln-Ser-NR₇R₈,
and cosmetically acceptable salts and solvates thereof,
wherein R₇ and R₈ are independently selected from H, methyl, ethyl, propyl and butyl.

A "dark spot on the skin" or "cutaneous dark spot" herein refers to hiperpigmentation of the skin caused by the increase of melanin. Hyperpigmentation can be diffuse or focal.

The "cosmetic treatment" of "dark spot on the skin" or "cutaneous dark spot" refers to lightening dark spots on the skin, eliminating (i.e. deleting) dark spots on the skin, reducing the size of dark spots on the skin and/or reducing the number of dark spots on the skin.

The "cosmetic prevention" of "dark spot on the skin" or "cutaneous dark spot" refers to prevent the appearance of dark spots on the skin, prevent the darkening of already existing dark spots on the skin, and /or prevent the increase of the size of already existing dark spots on the skin.

The term excipients or adjuvants also relate to carriers. Such pharmaceutical excipients, adjuvants or carriers can be liquids, such as water, oils or surfactants, including those of petroleum, animal, plant or synthetic origin, such as for example and in a non-limiting sense peanut oil, soybean oil, mineral oil, sesame oil, castor oils, polysorbates, sorbitan esters, ether sulfates, sulfates, betaines, glucosides, maltosides, fatty alcohols, nonoxinols, poloxamers, polyoxyethylenes, polyethylene glycols, dextrose, glycerol and the like. "Remington's Pharmaceutical Sciences" by E.W. Martin describes diluents, adjuvants or excipients as suitable carriers.

The following examples are merely illustrative of certain embodiments of the invention and cannot be considered as restricting it in any way.

### EXAMPLES

### Example 1. Melanocyte culture and incubation procedure

Day 1 - Human melanocytes are rise by trypsinization and centrifuge at 1500 rpm (433 x g) in an Allegra X-22R (Beckman Coulter) centrifuge during 5 minutes, and then are resuspended in 1 ml of a complete culture medium to count the number of cells. A volume of 10 µl of the cells solution was dyed with Trypan Blue colorant and the cells are counted with a hemocytometer. Then a cell solution is prepared at a concentration of 2,5x10⁵ cells/ml in a complete medium and 100 µl of the cell suspension is seed in wells of 96 well plates. The plates are incubated at 37°C in a 5% CO₂ atmosphere. Day 2 - The seed cells are washed and treated with the different products in basal conditions and promelanogenic conditions. For that, the medium is removed of wells and 100 ul of the products in basal medium with 0.5% of FBS is added. Previously, the test products are solubilised in the same medium. The plates are incubated 37 °C and 5% CO₂ during 5 days. The experiments are carried out using 4 wells per condition. The melanin accumulation inside the cells is monitored daily by optical microscopy.

Days 3 and 4 - All treatments are renewed every 24 hours.

Day 5 - Determination of cell viability: In order to determine de number of viable cells, 10 µl of CCK -8 (WST-8) is added in each well. The plates are incubated at 37 °C during 1 hour and the absorbance is measured at 450 nm using Synergy II multimode (Biotek Instruments Inc., Winooski, Estados Unidos). The mode and SD is calculated for each concentration using Microsoft Excel application.

### Example 2. Melanin determination

In order to determine the melanin content in cells treated as described in example 1, the medium is removed from each well and cells are washed with PBS 1N and incubated during 3 days at 37°C facilitating the solubilisation of the melanin in NaOH.

Day 7 - Once the solubilisation of melanin is complete, 50µl de H₂O₂ MilliQ is added per well and the content is determined by absorbance using a Synergy II (Biotek Instruments Inc., Winooski, USA) at 390 nm. The mode and SD is calculated for each concentration.

### Example 3. Melanine determination and mushroom tyrosinase activity inhibition

The activity of several peptides of formula (II) (listed in table 1 below) to reduce melanine was determined as explained in example 2 after treating the cells as explained in example 1.

The activity of several peptides of formula (II) to inhibit tyrosinase activity was determined as explained below:
The assay was performed in 96-well plates; 100µl of reaction mixture containing PBS pH 6.5, 25 µl of L-Tyrosine (1,5mM) and 20µl of the compounds at different concentrations were dispensed in each well. Then, 15 U of Mushroom Tyrosinase (20 µl) was added to each well. A reaction mixture without addition of any compound was also assayed and used as maximum activity control. Wells without enzyme served as reagent blank (Negative Control). The assay was performed using compound concentration range from 0 to 100µg/ml.

Plates were incubated at 30°C for 10 min and the amount of L-DOPA produced in the reaction mixture was measured using the Synergy II plate reader (Biotek) at 490 nm.

The activity was expressed as % of the maximum considered 100%.

**Table 1**

| **SEQUENCE** | **% INHIBITION TYROSINASE** | **% MELANINE CONTENT** |
|---|---|---|
| Ac-Ile-Ser-Leu-Leu-Asp-Ala-Gln-Ser-NH2 | 28 | 87 |
| Ac-Ile-Ser-Leu-Leu-Asp-(d)Ala-Gln-Ser-NH2 | 31 | 80 |
| Ac-Ile-Ser-(d)Leu-Leu-Asp-(d)Ala-Gln-Ser-NH2 | 40 | 68 |
| Palm-Ile-Ser-(d)Leu-Leu-Asp-(d)Ala-Gln-Ser-NH2 | 48 | 65 |
| Palm-Ile-Ser-Leu-Leu-Asp-(d)Ala-Gln-Ser-NH2 | 42 | 78 |
| Palm-Ile-Ser-Leu-Leu-Asp-Ala-Gln-Ser-NH2 | 39 | 85 |

## Claims

1. A peptide of formula (II):
R₅-Ile-Ser-Leu-Leu-Asp-Ala-Gln-Ser-R₆ R₅-[(SEQ ID NO: 2)-R₆] (II)
wherein
R₅ is selected from the group consisting of H-, R₇-C(O)- and R₇-OC(O)-,
R₆ is selected from the group consisting of -OH, -OR₇, -SR₇, and -NR₇R₈,
R₇ and R₈ are independently selected from H-, C₁-C₂₄ alkyl, C₂-C₂₄ alkenyl and C₆-C₁₀ aryl,
and cosmetically acceptable salts and solvates thereof.

2. Non-therapeutic use of the peptide of formula (II) as defined in claim 1 for the cosmetic prevention and/or cosmetic treatment of cutaneous dark spots.

3. A composition comprising a peptide of formula (II) as defined in claim 1 and one or more whitening agent selected from the group consisting of arbutine, vitamin C, ascorbyl phosphate, niacinamide, dynachondrine, a tyrosinase inhibitor peptide, tretinoin, hydroquinone, kojic acid and esters thereof with fatty acids, azelaic acid, glutathione, cinnamomum subavenium extracts, alpha-hydroxy acids such as lactic acid or glycolic acid, niacinamide, ferulic acid, vitamin E, ellagic acid, pomegrate extracts and mixtures thereof.

## Patentansprüche

1. Peptid gemäß Formel (II):
R₅-Ile-Ser-Leu-Leu-Asp-Ala-Gln-Ser-R₆ R₅-[(SEQ ID NO: 2)-R₆] (II)
wobei
R₅ ausgewählt ist aus der Gruppe bestehend aus H-, R₇-C(O)- und R₇-OC(O)-,
R₆ ausgewählt ist aus der Gruppe bestehend aus -OH-, -OR₇, -SR₇ und -NR₇R₈,
R₇ und R₈ unabhängig ausgewählt sind aus H-, C₁-C₂₄-Alkyl, C₂-C₂₄-Alkenyl und C₆-C₁₀-Aryl,
und kosmetisch akzeptable Salze und Solvate davon.

2. Nicht-therapeutische Verwendung des in Anspruch 1 definierten Peptids gemäß Formel (II) zur kosmetischen Prävention und/oder kosmetischen Behandlung von kutanen dunklen Flecken.

3. Zusammensetzung umfassend ein wie in Anspruch 1 definiertes Peptid gemäß Formel (II) und ein oder mehrere Aufhellungsmittel ausgewählt aus der Gruppe bestehend aus Arbutin, Vitamin C, Ascorbylphosphat, Niacinamid, Dinachondrine, einem Tyrosinaseinhibitor-Peptid, Tretinoin, Hydrochinon, Kojisäure und Estern davon mit Fettsäuren, Azelainsäure, Glutathion, Cinnamomum subavenium-Extrakten, alpha-Hydroxysäuren, wie Milchsäure oder Glycolsäure, Niacinamid, Ferulasäure, Vitamin E, Ellagsäure, Granatapfelextrakten und Mischungen davon.

## Revendications

1. Un peptide de formule (II) :
R₅-Ile-Ser-Leu-Leu-Asp-Ala-Gln-Ser-R₆ R₅-[(SEQ ID NO: 2)-R₆] (II)
où
R₅ est sélectionné parmi le groupe constitué de H-, R₇-C(O)- et R₇-OC(O)-,
R₆ est sélectionné parmi le groupe constitué de -OH, -OR₇, -SR₇, et -NR₇R₈,
R₇ et R₈ sont indépendamment sélectionnés parmi H-, alkyle en C₁-C₂₄, alcényle en C₂-C₂₄ et aryle en C₆-C₁₀,
et des sels et solvates cosmétiquement acceptables de celui-ci.

2. Utilisation non-thérapeutique du peptide de formula (II) tel que défini selon la revendication 1 pour la prévention cosmétique et/ou le traitement cosmétique de taches brunes cutanées.

3. Une composition comprenant un peptide de formule (II) tel que défini selon la revendication 1 et un ou plusieurs agents blanchissants sélectionnés parmi le groupe constitué d'arbutine, de vitamine C, de phosphate d'ascorbyle, de niacinamide, de dynachondrine, d'un peptide inhibiteur de tyrosinase, de trétinoïne, d'hydroquinone, d'acide kojique et d'esters de celui-ci avec des acides gras, d'acide azélaïque, de glutathion, d'extraits de cinnamomum subavenium, d'alpha-hydroxy acides comme l'acide lactique ou l'acide glycolique, de niacinamide, d'acide férulique, de vitamine E, d'acide ellagique, d'extraits de grenade et de mélanges de ceux-ci.
